# EUROPÄISCHE PATENTSCHRIFT

(11) **EP 1 272 492 B1**
(45) Veröffentlichungstag und Bekanntmachung des Hinweises auf die Patenterteilung: **06.10.2010**
(21) Anmeldenummer: 00958103.4
(22) Anmeldetag: 04.07.2000
(51) Int. Cl.: C07D 487/08, C07D 241/00

(54) **VERFAHREN ZUR HERSTELLUNG VON TRIETHYLENDIAMIN UNTER EINSATZ VON ETHYLENDIAMIN**
METHOD FOR PRODUCING TRIETHYLENE DIAMINE, USING ETHYLENE DIAMINE
PROCEDE DE PRODUCTION DE TRIETHYLENEDIAMINE AU MOYEN D'ETHYLENEDIAMINE

(30) Priorität: 05.07.1999 DE 19930736
(43) Veröffentlichungstag der Anmeldung: 08.01.2003
(73) Patentinhaber: BASF SE, 67056 Ludwigshafen (DE)
(72) Erfinder: KLOCKEMANN, Werner, D-21244 Buchholz (DE); FRAUENDORFER, Erich, D-26723 Emden (DE)
(86) Internationale Anmeldenummer: PCT/DE2000/002111
(87) Internationale Veröffentlichungsnummer: WO 2001/002404

(56) Entgegenhaltungen:
- EP-A- 0 313 753
- EP-A- 0 423 526
- WO-A-89/05810

## Beschreibung

Gegenstand der Erfindung ist ein Verfahren zur Herstellung von Triethylendiamin (1,4-Diazabicyclo[2.2.2]octan, im Folgenden TEDA genannt) unter Einsatz von Ethylendiamin (1,2-Diaminoethan, im Folgenden EDA genannt) und Zeolith-Katalysatoren vom Pentasil-Typ.

Triethlendiamin ist ein wichtiger chemischer Grundstoff und findet unter anderem Verwendung bei der Herstellung von Pharmazeutika und Kunststoffen, insbesondere als Katalysator bei der Herstellung von Polyurethanen.

Die bekannten Verfahren zur Herstellung von TEDA unterscheiden sich im wesentlichen durch die Art der Ausgangsprodukte und der Katalysatoren. Grundsätzlich ist es vorteilhaft als Edukte günstige Basischemikalien wie Ethanolamin oder Ethylendiamin (EDA) einzusetzen. Herkömmliche Verfahren haben sich jedoch, insbesondere gegenüber dem Edukt EDA, als sehr wenig selektiv erwiesen. Überdies ist die Abtrennung der Verunreinigungen die bei der Cyclisierungsreaktion entstehen schwierig, so daß sich diese Verfahren technisch nicht durchsetzen konnten.

Verfahren zur Herstellung von TEDA unter Verwendung von EDA sind bereits vorgeschlagen worden.

Das in der US 3,285,920 (H.G. Muhlbauer et al., Jefferson Chemical Co.) beschriebene Verfahren zur gleichzeitigen Herstellung von TEDA und Piperazin (im Folgenden PIP genannt) ist ein 2-Stufenprozess, nach dem man zunächst Ethylendiamin, Ethanolamin und/oder deren Oligomere in Gegenwart von Ammoniak, Wasserstoff zu einem Gemisch aus Piperazin und N-(Beta-aminoethyl)-piperazin in einem reduktiven Aminierungsverfahren unter Einsatz von metalloxidischen Hydrierkatalysatoren umsetzt und den verbleibenden Rest - nach Abtrennung des Piperazins - in Gegenwart von Cyclisierungskatalysatoren wie Phosphatsalzen und Alumosilikaten cyclisiert. Die Ausbeuten an TEDA liegen bei etwa 25 %, die von Piperazin bei etwa 12 %.

In der EP 0 158 319 (Union Carbide Corp.) werden Zeolith-Katalysatoren wie ZSM-5 zur Herstellung von TEDA vorgeschlagen. Im wesentlich hat die EP 0 158 319 cyclische Amin-Verbindungen als Edukte zum Gegenstand.

Aus der DE 37 35 212-A1 (entspricht EP 0 313 753 ) und der DE 37 35 214-A1 (entspricht EP 0 313 734, beide Hüls AG) ist ein Verfahren zur Herstellung eines PIP/TEDA Gemisches durch Umsetzung von Ethanolaminen und/oder Ethylendiamin in Gegenwart eines Zeolithen vom Pentasil-Typ bekannt. Nach dem Verfahren wird das Reaktionsgut bei 280 bis 380°C, einer LHSV (liquid hourly space velocity) 0,1 bis 10 h⁻¹ und bei einem Absolutdruck von 0,1 bis 10 bar in gasförmiger Form über einen Festbettkatalysator geleitet. Es wird auch vorgeschlagen, die Ausgangsverbindungen zusammen mit einem Verdünnungsmittel, wie z. B. Wasser, einzusetzen.

Gemäß der EP 0 382 055-A1 (entspricht DE 39 036 22, BASF AG) werden 1,2-Diaminoethan und 0 bis 200 mol% Piperazin an Aluminium-, Bor-, Gallium- und/oder Eisensilikat-Zeolithen bei folgenden bevorzugten Reaktionsbedingungen, im Falle einer Flüssigphasen-Reaktion, zu TEDA umgesetzt: Reaktionstemperatur 100 bis 300°C, Druck 1 bis 5 bar und WHSV 1 bis 10 h⁻¹. Soll die Reaktion in der Gasphase durchgeführt werden sind Temperaturen von 200 bis 400°C bevorzugt. Ein Lösungs- bzw. Verdünnungsmittel wie Wasser kann zugesetzt werden.

Die DE 39 34 459-A1 (Bayer AG) beschreibt ein Verfahren zur Herstellung von TEDA und Piperazin (PIP) durch Umsetzung von EDA an Zeolithen vom Pentasil-Typ mit abgeschwächter Acidität. Solche Zeolithe sind nach der DE 39 34 459-A1 durch Austausch mindestens 50% aller austauschbaren Kationen durch Alkalimetallkationen erhältlich oder sind solche, bei denen das Aluminium des Zeolith-Gerüstes isomorph durch Eisen ersetzt ist. Nicht nach diesen Verfahren behandelte ZSM-5 Katalysatoren haben sich gemäß der DE 39 34 459-A1 als weniger geeignet erwiesen. Die Umsetzung wird bei einer Temperatur von 300 bis 400 °C und bei einer Katalysatorbelastung von 0,03 bis 2,0 kg/kg EDA/kg Zeolith/h durchgeführt, wobei EDA-Wasser- Gemische mit 2 bis 25 Mol, vorzugsweise 5 bis 15 Mol, Wasser pro Mol EDA eingesetzt werden.

In der neueren U.S.- Patentliteratur (US 5,731,449 und US 5,741,906; Air Products and Chemicals Inc. bzw. US 5,041,548 Idemitsu Kosan Ltd.) werden Verfahren zur Herstellung von TEDA aus EDA unter Einsatz modifizierter Pentasil-Katalysatoren vorgeschlagen. Nach der US 5,041,548 (Idemitsu Kosan Ltd.) wird z.B. vorgeschlagen Zeolith-Katalysatoren des ZSM-5 -Typs einzusetzen, die in Gegenwart organischer Cyclisierungsmittel wie Tetraalkylammonium-Verbindungen hergestellt sind. In der US 5,756,741 (Air Products and Chemicals Inc.) wird ein zweistufiges Verfahren beschrieben, in dem zunächst aus einer Amino-Verbindung durch Cyclisierungsreaktion eine Piperazin reiche Zwischenstufe hergestellt wird, die dann unter Zusatz von z.B. EDA zu TEDA umgesetzt wird.

In der EP-A-0423526 wird ein Verfahren zur Herstellung von TEDA und Piperazin aus Ethylendiamin an Zeolith-Katalysatoren offenbart.

Die WO-A-89/05810 beschreibt ebenfalls ein Verfahren zur Herstellung von TEDA und/oder einem der mehreren Aminen durch Umsetzung eines oder mehrerer Amine an einem Zeolith-Katalysator.

Den Verfahren nach dem Stand der Technik ist eine geringe Selektivität im Hinblick auf die Bildung von TEDA, ein hoher Anteil an Wasser als Verdünnungsmittel und ggf. zusätzlich eine aufwendige Katalysatorherstellung / -modifizierung gemeinsam. Ziel der vorliegenden Erfindung ist es, ein Verfahren zur Herstellung von TEDA aus leicht zugänglichen N-haltigen Ausgangsverbindungen zu entwickeln, das einfach durchzuführen ist und vor allem einen gegenüber dem Stand der Technik erhöhte Selektivität gewährleisten soll. Gegenstand der Erfindung ist somit ein Verfahren zur Umsetzung von Ethylendiamin (EDA) unter Einsatz von Zeolith-Katalysatoren, das die Nachteile des Standes der Technik, wie insbesondere eine geringe Selektivität und hohen Piperazin-Anfall vermeidet und in hohen Ausbeuten zu TEDA von hoher Reinheit führt.

Die Aufgabe wird erfindungsgemäß gelöst durch ein Verfahren zur Herstellung von Triethylendiamin aus Ethylendiamin unter Einsatz von Zeolith-Katalysatoren, das dadurch gekennzeichnet ist, daß
- der Eduktstrom im kontinuierlichen Betrieb 5 bis 80 Gew.% Ethylendiamin, vorzugsweise 20 bis 70 Gew.% und besonders bevorzugt 35 bis 60 Gew.% enthält,
- die Zeolith-Katalysatoren Zeolith-Katalysatoren des Pentasil-Typs mit Si Al Atomverhältnissen von 150 zu 1 bis 700 zu 1, bevorzugt 150 zu 1 bis 350 zu 1, besonders bevorzugt 150 zu I bis 250 zu 1 und insbesondere von 150 zu 1 bis 250 zu 1, sind und zumindest teilweise in der H+ und/oder NH4+ - Form, vorzugsweise in der H+ - Form, vorliegen bzw. eingesetzt werden,
- der Eduktstrom einen Wassergehalt 2 bis 60 Gew.% , vorzugsweise 10 bis 40 Gew.%, besonders bevorzugt 10 bis 30 Gew.%, aufweist, bezogen auf den Eduktstrom, und
- die Reaktionstemperatur von 290 bis 400°C, bevorzugt 310 bis 370°C, besonders bevorzugt 310 bis 350 °C, beträgt.

Bevorzugte Ausführungsformen der Erfindung sind Gegenstand der Unteransprüche. Der Zeolith-Katalysator vom Pentasil-Typ, der als Katalysator in dem erfindungsgemäßen Verfahren zur Herstellung von Triethylendiamin eingesetzt wird, weist eine kristalline Skellet - Struktur aus Siliciumdioxid und Aluminiumoxid auf. Sofern der Zeolith-Katalysator vom Pentasil-Typ das Atomverhältnis Si : Al, wie oben angegeben, aufweist, bestehen im wesentlichen weder zusätzliche Erfordernisse bezüglich des Zeolith - Materials als solchem noch bezüglich des Verfahrens nach dem dieses erhältlich ist.

Als erfindungsgemäß einzusetzende Zeolith-Katalysatoren des Pentasil-Typs sind z.B. folgende Typen geeignet: ZSM-5, ZSM-11, ZSM-23, ZSM-53, NU-87, ZSM-35, ZSM-48 und Mischstrukturen aus mindestens zwei der oben genannten Zeolithe, insbesondere ZSM-5 und ZSM -11, sowie deren Mischstrukturen.

Liegt der erfindungsgemäß einzusetzende Zeolith-Katalysatoren des Pentasil-Typ aufgrund der Art der Produktion nicht in der gewünschten aciden H-Form vor, sondern z. B. in der Na-Form (oder einer anderen beliebigen Salzform), dann kann dieser durch Ionenaustausch, z. B. mit Ammoniumionen, und anschließender Kalzinierung oder durch Behandlung durch Säuren vollkommen oder partiell in die gewünschte H+ - oder NH₄+ -Form überführt werden. Um eine möglichst hohe Selektivität, hohe Umsätze sowie lange Standzeiten zu erreichen, kann es vorteilhaft sein, die Zeolithe zu modifizieren. Eine geeignete Modifizierung der Katalysatoren besteht darin, daß man das zeolithische Material - verformt oder unverformt - einer Behandlung, gemäß Stand der Technik, mit Protonen-Säuren - wie z. B. Salzsäure, Schwefelsäure, Flußsäure, Phosphorsäure, einer Carbonsäure oder Dicarbonsäure - und/oder Komplexbildnern und/oder Wasserdampf unterwirft.

Weiterhin haben sich folgende Reaktionsbedingungen als günstig erwiesen:
- eine WHSV (weight hourly space velocity) von 0,05 bis 6 h⁻¹, vorzugsweise von 0,2 bis 1 h⁻¹, besonders bevorzugt von 0,6 bis 1 h⁻¹ und
- ein Druck(absolut) von 0, 1 bis 10 bar, vorzugsweise von 0,8 bis 2 bar.

Der Eduktstrom kann mit einem inerten Trägergas, wie Stickstoff, und/oder Ammoniak angereichert sein und wird hierzu vorzugsweise mit Ammoniak gesättigt.

Vorzugsweise ist die Reaktion so zu führen, daß bei kontinuierlichem Betrieb im stationären Zustand Wasser, EDA und PIP im Gew.-Verhältnis von 10 bis 40 : 20 bis 60 : 20 bis 50, vorzugsweise von 10 bis 40 : 20 bis 50 : 20 bis 50, besonders bevorzugt von 15 bis 25 : 20 bis 50 : 20 bis 50, insbesondere etwa 20:40:40 zugeführt werden, wobei der Anteil des PIP oder des EDA ggf. zugunsten des einen bzw. zu Lasten des anderen erniedrigt bzw. erhöht werden kann. Es ist möglich auch andere Amin - Verbindungen in den Reaktor einzuschleusen und zu TEDA umzusetzen. Solche Amin - Verbindungen sind vorzugsweise Piperazin- Derivate wie z.B. Hydroxyethylpiperazin oder Aminoethylpiperazin. Der Zusatz dieser Verbindungen zum Eduktstrom sollte jedoch vorzugsweise einen Anteil von 20 Gew.% nicht überschreiten. 1,2-Ethanolamin kann ebenfalls eingesetzt werden. Da dieses jedoch zur Bildung von schwer abtrennbaren Nebenprodukten führen kann, ist es bevorzugt weniger als 5 Gew.% Ethanolamin einzusetzen.

Es hat sich gezeigt, daß bei einer Zugabe von insbesondere 35 bis 60 Gew.%, z.B. etwa 40 Gew.%, EDA die Reaktion kontinuierlich im stationären Zustand so geführt werden kann, daß sich EDA fast vollständig zu TEDA und PIP umsetzt, wobei PIP dem Produktstrom, z.B. destillativ, mit ggf. noch vorhandenen Zwischen- und/oder Nebenprodukten, entzogen wird und, etwa mit der selben Menge an EDA (in Gew.%) versetzt, der Reaktion erneut zugeführt werden kann.

Das Verfahren wird vorzugsweise durch Einstellung eines entsprechenden EDA / PIP Verhältnisses im Reaktorzulauf so durchgeführt, daß der Verbrauch an PIP in der Bilanz gegen 0 geht, d.h. im Ergebnis während des kontinuierlichen Betriebes im wesentlichen kein zusätzliches PIP zugeführt wird.

Bei einer solchen Reaktionsführung hat sich überraschend gezeigt, daß die Menge an ausgetragenen EDA gegen Null geht. Die Auftrennung des Reaktoraustrages ist nach dem erfindungsgemäßen Verfahren besonders einfach.

Ein besonderer Vorteil des Verfahrens besteht darin, daß man Zwischenfraktionen, die sowohl TEDA als auch Piperazin enthalten, erneut dem Katalysator zuführen kann. Weiterhin können Zwangsabfälle anderer Aminverbindungen aus anderen Amincyclisierungs-/ Kondensationsreaktionen der erfindungsgemäßen Reaktion zugeführt werden, ohne daß sich die Ausbeuten wesentlich verschlechtern.

Mit dem erfindungsgemäßen Verfahren werden somit folgende Vorteile erzielt:
- Das Verfahren erlaubt das als Edukt eingesetzte EDA je nach Preis und Verfügbarkeit durch Piperazin oder Piperazin - Derivate wie z.B. Hydroxyethylpiperazin oder Aminoethylpiperazin zu ersetzen.
- Es wird eine hohe Selektivität, bezogen auf die Umsetzung von EDA zu TEDA, erzielt.
- Das im wesentlichen einzige Nebenprodukt Piperazin kann bei geeigneter Reaktionsführung in den Prozeß erneut eingeschleust und dabei zu TEDA umgesetzt werden. Auch Gemische aus nicht umgesetztem Piperazin und TEDA können dem Katalysator erneut zugeführt werden, da sich gezeigt hat, daß TEDA unter den Reaktionsbedingungen stabil ist.

Zur Erhöhung der Standfestigkeit können die erfindungsgemäß einzusetzenden Zeolithe geträgert werden z.B. auf Cellulosematerialien, Tonen, Bindemitteln oder Metalloxiden wie Tonerden, Aluminiumoxid, Siliciumdioxid. Weiterhin ist es möglich diese als Granulat, in Kugelform oder auf Glas- oder andere Körper aufgebracht einzusetzen.

Als verfestigende Formgebungsprozesse für die erfindungsgemäß einzusetzenden Zeolithe können im Prinzip alle Methoden zur Erlangung einer entsprechenden Formung verwendet werden. Bevorzugt werden Verfahren, bei denen die Formgebung durch Extrusion in üblichen Extrudern, beispielsweise zu Strängen mit einem Durchmesser von üblicherweise 1 bis 10 mm, insbesondere 2 bis 5 mm, erfolgt. Werden Bindemittel und/oder Hilfsmittel benötigt, ist der Extrusion zweckmäßigerweise ein Mischungs- oder Knetprozeß vorgeschaltet. Gegebenenfalls erfolgt nach der Extrusion noch ein Kalcinierungsschritt. Die erhaltenen Stränge werden gewünschtensfalls zerkleinert, vorzugsweise zu Granulat oder Splitt mit einem Partikeldurchmesser von 0,5 bis 5 mm, inbesondere 0,5 bis 2 mm. Dieses Granulat oder dieser Splitt und auch auf anderem Wege erzeugte Katalysatorformkörper enthalten praktisch keine feinkörnigeren Anteile als solche mit 0,5 mm Mindestpartikeldurchmesser.

In einer bevorzugten Ausführungsform enthält der geformte, erfindungsgemäß einzusetzende Zeolith bis zu 80 Gew.% Bindemittel, bezogen auf die Gesamtmasse des Katalysators. Besonders bevorzugte Bindemittelgehalte sind 1 bis 50 Gew.%, insbesondere 3 bis 35 Gew.%. Als Bindemittel eignen sich im Prinzip alle für derartige Zwecke eingesetzte Verbindungen, bevorzugt werden Verbindungen, inbesondere Oxide, des Siliciums, Aluminiums, Bors, Phosphors, Zirkoniums und/oder Titans. Von besonderem Interesse als Bindemittel ist Siliciumdioxid, wobei das SiO₂ auch als Kieselsol oder in Form von Tetraalkoxysilanen in den Formgebungsprozeß eingebracht werden kann. Auch als Bindemittel verwendbar sind Oxide des Magnesiums und Berylliums sowie Tone, z.B. Montmorillonit, Kaoline, Bentonite, Halloysite, Dickite, Nacrite und Anauxite.

Als Hilfsmittel für die verfestigenden Formgebungsprozesse sind beispielsweise Verstrangungshilfsmittel für die Extrusion zu nennen, ein übliches Verstrangungsmittel ist Methylcellulose. Derartige Mittel werden in der Regel in einem nachfolgendem Calcinierungsschritt vollständig verbrannt.

Bei Verwendung des erfindungsgemäß einzusetzenden Zeolith-Katalysators kann dieser unabhängig von seiner Form nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet werden, die genau definierte Mengen an Sauerstoff-liefernden Substanzen enthält. Ein solches Regenerierungsverfahren ist unter anderem in der WO 98/55228 bzw. der DE 19723949-A1 beschrieben, deren Offenbarung durch diesbezügliche Bezugnahme hiermit vollumfänglich auch zum Gegenstand der vorliegenden Anmeldung gemacht wird.

Der zu regenerierende, erfindungsgemäß einzusetzende Zeolith-Katalysator wird entweder in der Umsetzungsvorrichtung oder in einem externen Ofen in einer Atmosphäre, die 0,1 bis ungefähr 20 Volumen-Anteile von Sauerstoff-liefernden Substanzen, besonders bevorzugt Sauerstoff, enthält, auf eine Temperatur im Bereich von ungefähr 250 °C bis 800 °C, vorzugsweise ungefähr 400 °C bis 550 °C und insbesondere ungefähr 450 °C bis 500 °C aufgeheizt. Dabei wird das Aufheizen vorzugsweise mit einer Aufheizrate von ungefähr 0,1 °C/min. bis ungefähr 20°C/min., vorzugsweise ungefähr 0,3°C/min bis ungefähr 15°C/min. und insbesondere 0,5°C/min. bis 10°C/min. durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, daß es zu Schädigungen der Katalysatorstruktur kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators.

Sinkt die Temperatur des Abgasstroms am Reaktorausgang trotz steigender Mengen an Sauerstoffliefernden Substanzen im Gasstrom, so ist das Abbrennen der organischen Beläge beendet. Die Dauer der Behandlung beträgt im allgemeinen jeweils ungefähr 1 bis 30, vorzugsweise ungefähr 2 bis ungefähr 20 und insbesondere ungefähr 3 bis ungefähr 10 Stunden.

Beim anschließenden Abkühlen des so regenerierten Katalysators ist darauf zu achten, daß das Abkühlen nicht zu schnell erfolgt ("Abschrecken"), da sonst die mechanische Festigkeit des Katalysator negativ beeinflußt werden kann.

Der obige Begriff "Sauerstoff-liefernde Substanzen" umfasst alle Substanzen, die in der Lage sind, unter den angegebenen Regenerierungsbedingungen Sauerstoff abzugeben oder kohlenstoff- und/oder stickstoffhaltige Rückstände zu entfernen. Insbesondere zu nennen sind: Stickoxide der Formel NₓO_{y}, wobei x und y so gewählt werden, daß sich ein neutrales Stickoxid ergibt, N₂0, N₂0 haltiger Abgasstrom aus einer Adipinsäureanlage, NO, N0₄, Ozon oder ein Gemisch aus zwei oder mehr davon. Bei Verwendung von CO₂ als Sauerstoff -liefernde Substanz werden bevorzugt Temperaturen von 500 °C bis 800 °C während der Regeneration eingestellt.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, daß zwar die jeweils am Katalysator anhaftenden Wertprodukte von diesem entfernt werden können, aber Temperatur und Druck nicht so hoch gewählt werden, daß die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Um-setzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere in den Mikroporen zu vermeiden, da auch dies zu Schädigungen des Katalysators führen kann.

Eine bevorzugte Ausführung des Herstellprozesses kann darin bestehen, daß das erfindungsgemäße, kontinuierliche Verfahren zur Synthese von TEDA bei der Regeneration des erfindungsgemäßen Katalysators nicht unterbrochen werden muß, um den Verfahrensdurchsatz zu steigern. Dies kann durch die Verwendung von mindestens zwei parallel verschalteten Reaktoren erreicht werden.

Die Katalysatorregeneration kann derart durchgeführt werden, daß mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung von EDA zur Verfügung steht.

Ggf. können neben Wasser auch andere Verdünnungsmittel wie etwa Stickstoff oder Ammoniak (z.B. über die Sättigung des Eduktstroms hinaus) dem Katalysator zugeführt werden. Die Eduktkomponenten werden vorteilhafterweise vortemperiert.

Die Aufarbeitung des Reaktionsgemisches kann nach üblichen Methoden erfolgen. Eine besonders zweckmäßige Form besteht darin, das Reaktionsgemisch aufzufangen und destillativ aufzutrennen. Sofern gewünscht, kann TEDA zur Verbesserung seiner Reinheit aus geeigneten Lösemitteln umkristallisiert werden. Meist ist dies jedoch nicht erforderlich, da TEDA nach dem erfindungsgemäßen Verfahren mit Reinheiten größer 95 Gew.% hergestellt werden kann.

### Versuchsbeispiel

In einem Festbettreaktor (1 cm Innendurchmesser, 1 m Länge) wurde die Reaktion unter isothermen Bedingungen bei 320 °C für größer 24 h durchgeführt. Die Reaktionsprodukte wurden durch übliche Methoden abgetrennt und charakterisiert. Die quantitative Bestimmung der Reaktionsprodukte erfolgte gaschromatographisch.

Als Katalysator wurde ein ZSM-5 Zeolith verwendet (SiO/Al₂O₃ molares Verhältnis von 400 entsprechend einem Atomverhältnis Si:Al von 200, H+ Form ), der vor dem Verstrangen mit 20 Gew.% Si0₂ (bezogen auf die Gesamtmasse der fertigen Stränge) mit 5%-iger HCl gewaschen wurde.

Das Einsatzprodukt bestand aus 16% Wasser, 36% Ethylendiamin, 36% Piperazin und 12% Triethylendiamin. Der Eduktstrom wird so eingestellt, daß sich eine Katalysatorbelastung (WHSV, weight hourly space velocity, g Eduktmischung/ g Katalysator/ h ) von 0,6 h bis 1 h ergab. Der Umsatz von Ethylendiamin betrug 100% und die Ausbeute an Triethylendiamin 97,5%.

Produkt- bzw. Eduktgemisch wiesen bei Beispielsansätzen, die etwa im stationären Zustand gefahren wurden, etwa die folgende Zusammensetzungen auf (jeweils in Gew.%)
Eduktgemisch
   H₂0 15, EDA 36; PIP 36,0; TEDA 12; Nebenprodukte 1
Produktgemisch
   H₂0 17, EDA - ; PIP 41 ; TEDA 38; Nebenprodukte 3
Als Nebenproduke / Zwischenprodukte wurden folgende Verbindungen detektiert:
   Dimethyl-pyrazine 0,4; N-Ethyl-piperazin 0,4; DETA 0,2
   NAEP 1,0; höhere Oligomere der EPI (Sdp. >200°C) 1,3.

## Patentansprüche

1. Verfahren zur Herstellung von Triethylendiamin aus Ethylendiamin unter Einsatz von Zeolith-Katalysatoren **dadurch gekennzeichnet, dass**
- der Eduktstrom in kontinuierlichen Betrieb 5 bis 80 Gew.% Ethlendiamin enthält
- die Zeolith-Katalysatoren Zeolith-Katalysatoren des Pentasil-Typs mit Si : Al A-tomverhältnissen von 150 zu 1 bis 700 zu 1 sind und zumindest teilweise in der H+ und/oder NH₄+ - Form vorliegen bzw. eingesetzt werden,
- der Eduktstrom einen Wassergehalt 2 bis 60 Gew.% aufweist und
- die Reaktionstemperatur von 290 bis 400°C beträgt.

2. Verfahren gemäß Anspruch 1, **dadurch gekennzeichnet, dass** die Zeolith-Katalysatoren Si : Al Atomverhältnisse von 150 zu 1 bis 250 zu 1 aufweisen.

3. Verfahren gemäß einem der vorhergehenden Ansprüche, **dadurch gekennzeichnet, dass** die Reaktionstemperatur von 310 bis 370°C beträgt.

4. Verfahren gemäß einem der vorhergehenden Ansprüche, **dadurch gekennzeichnet, dass** der Eduktstrom in kontinuierlichen Betrieb 20 bis 70 Gew.% Ethylendiamin enthält.

5. Verfahren gemäß einem der vorhergehenden Ansprüche, **dadurch gekennzeichnet, dass** die WHSV von 0,05 bis 6 h¹ beträgt.

6. Verfahren gemäß einem der vorhergehenden Ansprüche, **dadurch gekennzeichnet, dass** der Druck (absolut) im Reaktor von 0,1 bis 10 bar beträgt.

7. Verfahren gemäß einem der vorhergehenden Ansprüche, **dadurch gekennzeichnet, dass** Ethylendiamin zusammen mit 0 bis 200 Mol % Piperazin eingesetzt wird.

8. Verfahren gemäß einem der vorhergehenden Ansprüche, **dadurch gekennzeichnet, dass** Wasser / Ethylendiamin / Piperazin im Eduktstrom etwa im Gewichtsverhältnis 10 bis 40 : 20 bis 50 : 20 bis 50 eingesetzt werden.

9. Verfahren gemäß einem der vorhergehenden Ansprüche, **dadurch gekennzeichnet, dass** die Zeolith-Katalysatoren vom Pentasil-Typ Zeolithe vom Typ ZSM-5, ZSM-11 oder deren Mischstrukturen sind.

10. Verfahren gemäß einem der vorhergehenden Ansprüche, **dadurch gekennzeichnet, dass** zumindest teilweise Zeolith-Katalysatoren eingesetzt werden, die in einer Inertgas- Atmosphäre in Gegenwart von Sauerstoff oder Sauerstoff-liefernden Substanzen bei einer Temperatur im Bereich von 250°C bis 800°C regeneriert wurden, vorzugsweise mit einer Aufheizrate von etwa 0,1 °C/min. bis etwa 20°C/min.

11. Verfahren gemäß einem der vorhergehenden Ansprüche, **dadurch gekennzeichnet, dass** die Umsetzung ohne Unterbrechung kontinuierlich in mindestens zwei parallel verschalteten Reaktionsräumen geführt wird, von denen zumindest einer zur Regeneration des Katalysators vom Edukt- und / oder Produktstrom abkoppelbar ist.

12. Verfahren gemäß einem der vorhergehenden Ansprüche, **dadurch gekennzeichnet, dass** die Zeolith-Katalysatoren vom Pentasil-Typ vor dem Einsatz in dem Verfahren, vorzugsweise vor Aufbringen auf einem Träger und / oder Verstrangen, mit einer Proton-Säure gewaschen werden.

13. Verfahren zur Herstellung von Triethylendiamin aus Ethylendiamin unter Einsatz von Zeolith-Katalysatoren **dadurch gekennzeichnet, dass**
- der Eduktstrom im kontinuierlichen Betrieb 5 bis 80 Gew.% Ethylendiamin enthält,
- die Zeolith-Katalysatoren Zeolith-Katalysatoren des Pentasil-Typs mit Si : Al Atomverhältnissen von 150 zu 1 bis 700 zu 1 sind und zumindest teilweise in der H+ und/oder NH₄ + - Form vorliegen bzw. eingesetzt werden,
- der Eduktstrom einen Wassergehalt 2 bis 60 Gew.% aufweist,
- die Reaktionstemperatur von 290 bis 400°C beträgt, und dass
- Wasser / Ethylendiamin / Piperazin im Eduktstrom etwa im Gewichtsverhältnis 10 bis 40 : 20 bis 50 : 20 bis 50 eingesetzt werden.

## Claims

1. A process for preparing triethylenediamine from ethylenediamine using zeolite catalysts, wherein
- the feed stream in continuous operation comprises from 5 to 80% by weight of ethylenediamine
- the zeolite catalysts are zeolite catalysts of the pentasil type having Si:Al atomic ratios of from 150:1 to 700:1 and are present or used at least partly in the H⁺ and/or NH₄⁺ form,
- the feed stream has a water content of from 2 to 60% by weight and
- the reaction temperature is from 290 to 400°C.

2. The process according to claim 1, wherein the zeolite catalysts have Si:Al atomic ratios of from 150:1 to 250:1.

3. The process according to either of the preceding claims, wherein the reaction temperature is from 310 to 370°C.

4. The process according to any of the preceding claims, wherein the feed stream in continuous operation comprises from 20 to 70% by weight of ethylenediamine.

5. The process according to any of the preceding claims, wherein the WHSV is from 0.05 to 6 h⁻¹.

6. The process according to any of the preceding claims, wherein the pressure (absolute) in the reactor is from 0.1 to 10 bar.

7. The process according to any of the preceding claims, wherein ethylenediamine is used together with from 0 to 200 mol% of piperazine.

8. The process according to any of the preceding claims, wherein water/ethylenediamine/piperazine are used in the feed stream in an approximate weight ratio of 10-40:20-50:20-50.

9. The process according to any of the preceding claims, wherein the zeolite catalysts of the pentasil type are zeolites of the ZSM-5 type, the ZSM-11 type or mixed structures derived therefrom.

10. The process according to any of the preceding claims, wherein at least part of the zeolite catalysts used are zeolite catalysts which have been regenerated in an inert gas atmosphere in the presence of oxygen or oxygen-supplying substances at a temperature in the range from 250°C to 800°C, preferably at a heating rate of from about 0.1°C/min to about 20°C/min.

11. The process according to any of the preceding claims, wherein the reaction is carried out continuously without interruption in at least two reaction spaces connected in parallel, of which at least one can be decoupled from the feed stream and/or product stream in order to regenerate the catalyst.

12. The process according to any of the preceding claims, wherein the zeolite catalysts of the pentasil type are washed with a protic acid before use in the process, preferably before application to a support and/or extrusion.

13. A process for preparing triethylenediamine from ethylenediamine using zeolite catalysts, wherein
- the feed stream in continuous operation comprises from 5 to 80% by weight of ethylenediamine
- the zeolite catalysts are zeolite catalysts of the pentasil type having Si:Al atomic ratios of from 150:1 to 700:1 and are present or used at least partly in the H⁺ and/or NH₄⁺ form,
- the feed stream has a water content of from 2 to 60% by weight,
- the reaction temperature is from 290 to 400°C and
- water/ethylenediamine/piperazine are used in the feed stream in a weight ratio of about 10-40:20-50:20-50.

## Revendications

1. Procédé pour la préparation de triéthylènediamine à partir d'éthylènediamine avec utilisation de catalyseurs zéolithiques, **caractérisé en ce que**
- le flux de produits de départ contient, en fonctionnement continu, 5 à 80% en poids d'éthylènediamine
- les catalyseurs zéolithiques sont des catalyseurs zéolithiques du type Pentasil avec des rapports atomiques Si:Al de 150:1 à 700:1 et se trouvent ou sont utilisés au moins partiellement sous la forme H⁺ et/ou NH₄⁺,
- le flux de produits de départ présente une teneur en eau de 2 à 60% en poids et
- la température de réaction est de 290°C à 400°C.

2. Procédé selon la revendication 1, **caractérisé en ce que** les catalyseurs zéolithiques présentent des rapports atomiques Si:Al de 150:1 à 250:1.

3. Procédé selon l'une quelconque des revendications précédentes, **caractérisé en ce que** la température de réaction est de 310°C à 370°C.

4. Procédé selon l'une quelconque des revendications précédentes, **caractérisé en ce que** le flux de produits de départ contient, en fonctionnement continu, 20 à 70% en poids d'éthylènediamine.

5. Procédé selon l'une quelconque des revendications précédentes, **caractérisé en ce que** le WHSV (weight hourly space velocity - vitesse horaire spatiale en poids) est de 0,05 à 6 h⁻¹.

6. Procédé selon l'une quelconque des revendications précédentes, **caractérisé en ce que** la pression (absolue) dans le réacteur est de 0,1 à 10 bars.

7. Procédé selon l'une quelconque des revendications précédentes, **caractérisé en ce que** l'éthylènediamine est utilisée ensemble avec 0 à 200% en mole de pipérazine.

8. Procédé selon l'une quelconque des revendications précédentes, **caractérisé en ce que** l'eau/l'éthylènediamine/la pipérazine sont utilisées dans le flux de produits de départ dans un rapport pondéral d'environ 10 à 40:20 à 50:20 à 50.

9. Procédé selon l'une quelconque des revendications précédentes, **caractérisé en ce que** les catalyseurs zéolithiques du type Pentasil sont des zéolithes du type ZSM-5, ZSM-11 ou leurs structures mixtes.

10. Procédé selon l'une quelconque des revendications précédentes, **caractérisé en ce qu'**on utilise au moins partiellement des catalyseurs zéolithiques qui ont été régénérés dans une atmosphère de gaz inerte en présence d'oxygène ou de substances fournissant de l'oxygène à une température dans la plage de 250°C à 800°C, de préférence à une vitesse de chauffage d'environ 0,1°C/min. jusqu'à environ 20°C/min.

11. Procédé selon l'une quelconque des revendications précédentes, **caractérisé en ce que** la transformation est réalisée sans interruption, en continu dans au moins deux espaces de réaction commutés en parallèle, dont au moins un peut être découplé du flux de produits de départ et/ou de produits pour la régénérateur du catalyseur.

12. Procédé selon l'une quelconque des revendications précédentes, **caractérisé en ce que** les catalyseurs du type Pentasil sont lavés avant l'utilisation dans le procédé, de préférence avant l'application sur un support et/ou la formation de brins, avec un acide protonique.

13. Procédé pour la préparation de triéthylènediamine à partir d'éthylènediamine avec utilisation de catalyseurs zéolithiques, **caractérisé en ce que**
- le flux de produits de départ contient, en fonctionnement continu, 5 à 80% en poids d'éthylènediamine,
- les catalyseurs zéolithiques sont des catalyseurs zéolithiques du type avec des rapports atomiques Si:Al de 150:1 à 700:1 et se trouvent ou sont utilisés au moins partiellement sous la forme H⁺ et/ou NH₄⁺,
- le flux de produits de départ présente une teneur en eau de 2 à 60% en poids
- la température de réaction est de 290°C à 400°C et **en ce que**
- l'eau/l'éthylènediamin/la pipérazine sont utilisées dans le flux de produits de départ dans un rapport pondéral d'environ 10 à 40:20 à 50:20 à 50.
